# EUROPEAN PATENT SPECIFICATION

(11) **EP 4 482 421 B1**
(45) Date of publication and mention of the grant of the patent: **02.09.2026**
(21) Application number: 23710792.5
(22) Date of filing: 20.02.2023
(51) Int. Cl.: A61B 50/13, A61M 1/36, A61B 50/18

(54) **EQUIPMENT FOR THE TRANSPORT OF BIOMEDICAL DEVICES**
VORRICHTUNG ZUM TRANSPORT VON BIOMEDIZINISCHEN VORRICHTUNGEN
ÉQUIPEMENT DE TRANSPORT DE DISPOSITIFS BIOMÉDICAUX

(30) Priority: 24.02.2022 IT 202200003455
(43) Date of publication of application: 01.01.2025
(73) Proprietor: Eurosets S.r.l., 41036 Medolla (MO) (IT)
(72) Inventor: FONTANILI, Paolo, 41036 Medolla (MO) (IT); PETRALIA, Antonio, 41036 Medolla (MO) (IT); ZERBINI, Alessandro, 41036 Medolla (MO) (IT)
(74) Representative: Zoli, Filippo
(86) International application number: PCT/IB2023/051536
(87) International publication number: WO 2023/161785

(56) References cited:
- WO-A1-2020/152515
- WO-A2-2012/158560
- CN-A- 113 288 751
- CN-U- 208 574 062

## Description

### Technical Field

The present invention relates to a piece of equipment for the transport of biomedical devices.

### Background Art

Equipment is known to transport, both in and out of the hospital, biomedical devices, such as extracorporeal blood circulation devices, e.g., oxygenators and centrifugal pumps, or other devices used in hospital practice and in emergency situations.

These known types of equipment are generally of the trolley type or the like, i.e., they comprise a load-bearing structure associated with means of movement, of the wheel type, and supporting a resting plane for one or more biomedical devices.

These known types of equipment do have some drawbacks.

Since they have to support a plurality of devices which must be interconnected in order to enable the proper operation of extracorporeal circulation, they in fact do not allow for easy and practical maintenance of the same.

In order to carry out the necessary cleaning and maintenance operations of the devices placed on the equipment of known type, operators must in fact move the devices themselves, removing the connections in place and locating them elsewhere so that they can conveniently work on them. This limitation is particularly clear in maintenance operations on the heat exchanger which is connected to the oxygenator and which must undergo regular cleaning operations to eliminate any bacteria that may lurk inside and which can be highly harmful to the patient undergoing extracorporeal circulation.

As can be easily guessed, the removal of the devices to be maintained from the relevant transport equipment is not easy and involves non-negligible downtime.

Alternatively, operators can also carry out the necessary maintenance operations by keeping the devices in place on the relevant equipment.

This solution, too, has drawbacks however, since the space available for operators to work is particularly small so that access to the devices is particularly difficult and uneasy.

Still another drawback is the difficulty of conveniently and easily placing all the auxiliary devices necessary for the operation of extracorporeal circulation, since the transport equipment of known type has small overall dimensions constrained by hospital requirements.

Equipment for the transport of medical devices are known from WO 2020/152515 A1 and WO 2012/158560 A1.

### Description of the Invention

The main aim of the present invention is to devise a piece of equipment for the transport of biomedical devices which enables easy maintenance of the various devices being transported.

Within this aim, one object of the present invention is to simplify maintenance operations without the need to remove the devices from the relevant equipment. Another object of the present invention is to enable easy and convenient placement and connection of the various devices necessary for the operation of extracorporeal circulation.

Another object of the present invention is to devise a piece of equipment for the transport of biomedical devices which can overcome the aforementioned drawbacks of the prior art within the framework of a simple, rational, easy and effective to use as well as affordable solution.

The aforementioned objects are achieved by this piece of equipment for the transport of biomedical devices according to claim 1.

### Brief Description of the Drawings

Other characteristics and advantages of the present invention will become more apparent from the description of a preferred, but not exclusive, embodiment of a piece of equipment for the transport of biomedical devices, illustrated by way of an indicative, yet non-limiting example, in the accompanying tables of drawings in which:

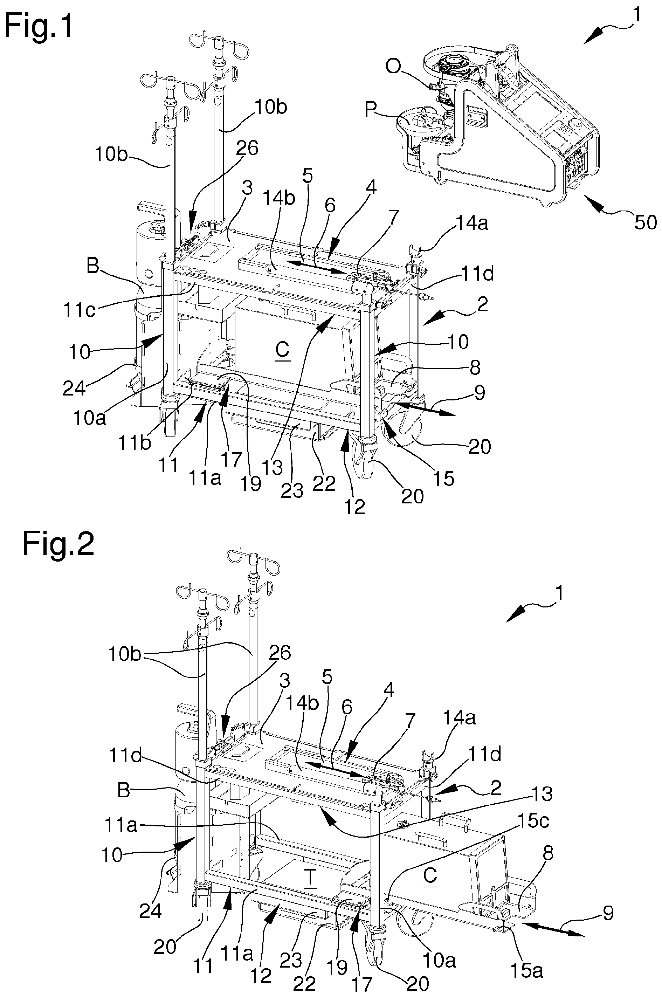

Figure 1 is an axonometric view of a piece of equipment for the transport of biomedical devices according to the invention with the intermediate plane in the working position;
Figure 2 is a view of the equipment in Figure 1 with the intermediate plane in the maintenance position;
Figure 3 is a sectional view of the equipment in Figure 1;
Figure 4 is a magnifying view of a detail of the equipment in Figure 3;

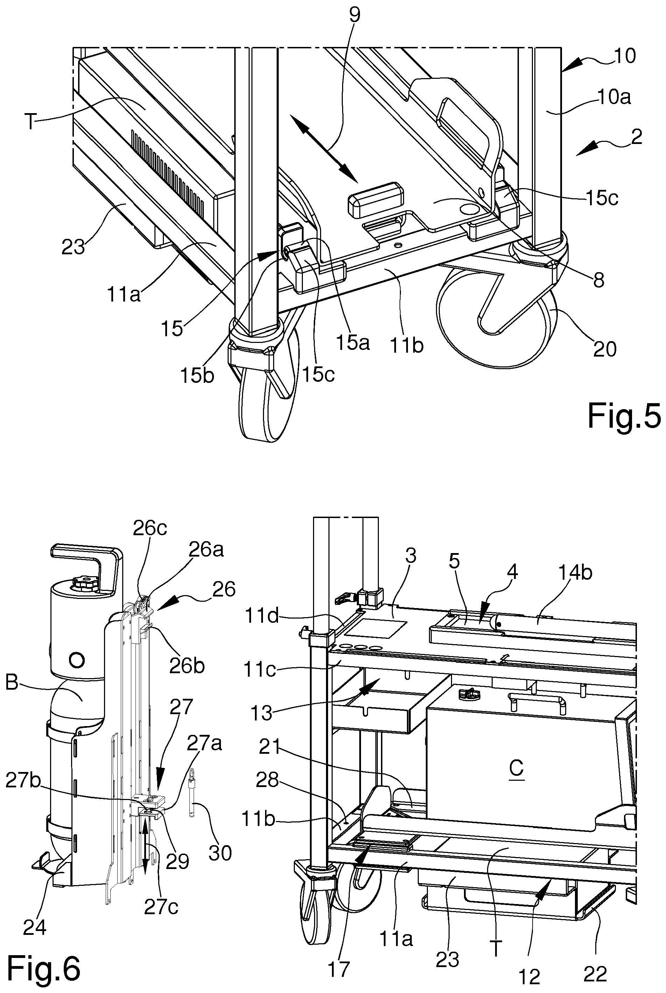

Figure 5 is a magnifying view of a detail of the equipment in Figure 1, without the heat exchanger;
Figure 6 is a partial exploded view of the equipment in Figure 1;

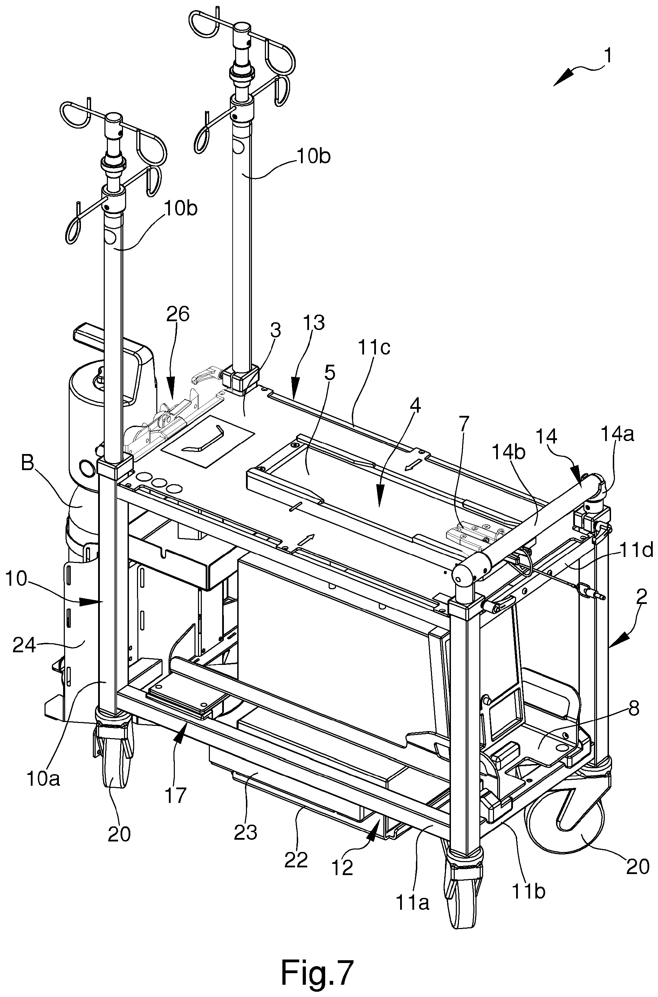

Figure 7 is an axonometric view of the equipment in Figure 1 with the movable portion of the gripping element in the transport position.

### Embodiments of the Invention

With particular reference to these figures, reference numeral 1 globally indicates a piece of equipment 1 for the transport of biomedical devices.

The equipment 1 comprises a load-bearing framing 2, movement means 20 of the framing 2 with respect to a resting surface S, at least one supporting plane 3 associated with the framing 2 and provided with removable anchoring means 4 of at least one biomedical device O, P.

More particularly, at least one piece of equipment for the support of biomedical devices 50 such as, e.g., an oxygenator O and the relevant pumping assembly P for extracorporeal blood circulation can be positioned on the supporting plane 3. Specifically, the anchoring means 4 comprise at least one guiding element 5 associated with the supporting plane 3 and developing along one direction of insertion/extraction 6 and removable retaining means 7 associated with the guiding element itself. The guiding element 5 is engageable by shifting along the direction of insertion/extraction 6 by an anchoring element (not shown in detail in the figures) which can be associated with the equipment for the support of biomedical devices 50 and the retaining means 7 are adapted to clamp the anchoring element itself along the direction of insertion/extraction 6 upon reaching a predefined anchoring position.

According to the invention, the equipment 1 also comprises an intermediate plane 8, for the support of an additional device, e.g. of the type of a heat exchanger C, intended to be connected to the biomedical device O, P. The intermediate plane 8 is associated movable by shifting with the framing 2 along a direction of sliding 9 between a working position, wherein it is positioned inferiorly to the supporting plane 3, and a maintenance position, wherein it is shifted with respect to the working position along the direction of sliding 9 so as to arrange itself at least partly beyond the overall dimensions defined by the framing itself. In the maintenance position, the intermediate plane 8 is associated cantilevered with the framing 2.

Preferably, the intermediate plane 8 is substantially vertically aligned with the supporting plane 3 in the working position and is vertically offset with respect to the supporting plane 3 in the maintenance position.

Appropriately, the intermediate plane 8 is associated with the framing 2 in the maintenance position.

More particularly, the intermediate plane 8 is constrained to the framing 2 in the maintenance position.

The intermediate plane 8, in the maintenance position, thus has an extremity portion associated with the framing 2 and the opposite extremity portion arranged outside of the framing 2 and disengaged therefrom.

When the intermediate plane 8 is brought to the maintenance position, it is then easily accessible by operators to carry out maintenance operations, such as sterilizing the heat exchanger C in order to eliminate any bacteria inside it. Once maintenance operations are completed, the intermediate plane 8 can be returned to the working position.

More particularly, the framing 2 comprises a plurality of load-bearing elements 10,11 having an elongated shape and associated with each other, of which a plurality of vertical elements 10 and a plurality of horizontal elements 11 interconnected with each other.

In the particular embodiment shown in the figures, the framing 2 consists of at least four vertical elements 10 and a plurality of horizontal elements 11 locked together with the vertical elements 10. In this description, the terms "vertical" and "horizontal" refer to the arrangement taken by the elements under the conditions of normal use of the equipment 1, i.e., with the movement means 20 resting on the resting surface S, such as the ground.

Appropriately, at least two of the vertical elements 10 comprise a fixed body 10a and a movable body 10b telescopically fitted within the relevant fixed body 10a. The movable body 10b can then be moved in a sliding manner within the fixed body 10a so as to adjust its height with respect to the resting surface S. Preferably, as in the embodiment shown in the figures, all the vertical elements 10 of the framing 2 comprise a relevant fixed body 10a and a relevant movable body 10b.

The movement means 20 are, e.g., of the type of swivel wheels which are associated with the vertical elements 10.

Advantageously, the framing 2 comprises at least a first lattice 12 supporting the intermediate plane 8 and provided with a pair of first longitudinal elements 11a, which develop parallel to the direction of sliding 9, and with a pair of first transverse elements 11b, which develop transverse to the longitudinal elements 11a.

In more detail, the longitudinal elements 11a are arranged substantially (i.e., unless machining tolerances) perpendicular to the transverse elements 11b and show greater extension than them. It follows, therefore, that the first lattice 12 has quadrangular conformation, elongated in the direction of sliding 9.

The framing 2 therefore has elongated conformation and the term "longitudinal", used in this description with reference to the horizontal elements 11, therefore identifies that these elements develop in the direction of the length of the framing itself.

As can be seen from the figures, the framing 2 also comprises a second lattice 13 supporting the supporting plane 3 and provided with a pair of second longitudinal elements 11c, which develop parallel to the direction of sliding 9 and with a pair of second transverse elements 11d, arranged transverse to the second longitudinal elements 11c.

In more detail, again the second longitudinal elements 11c are arranged substantially perpendicularly to the second transverse elements 11d.

In other words, the first longitudinal elements 11a and the second longitudinal elements 11c are arranged parallel to each other and the first transverse elements 11b and the second transverse elements 11d are arranged parallel to each other.

Advantageously, the equipment 1 comprises removable clamping means 15 of the intermediate plane 8 with respect to the framing 2 in the working position.

More particularly, the clamping means 15 comprise at least one pair of clamping elements 15a locked together with the intermediate plane 8 and at least one pair of seats 15b associated with the framing 2, wherein the seats 15b are adapted to receive the clamping elements 15a in the working position of the intermediate plane 8, preventing the movement thereof along the direction of sliding 9. The seats 15b then define relevant abutment walls for the clamping elements 15a. The clamping elements 15a are liftable with respect to the seats 15b, so that they can be carried outside the seats themselves and then be moved towards the maintenance position.

In addition, the clamping means 15 also comprise a pair of ascent elements 15c adapted to guide the clamping elements 15a within the seats 15b upon completion of the displacement of the intermediate plane 8 from the maintenance position to the working position. In actual facts, the ascent elements 15c define respective inclined surfaces adapted to lift the intermediate plane 8 to allow it to enter the inside of the seats 15b.

Appropriately, the seats 15b are defined at one of the first transverse elements 11b, and the clamping elements 15a are arranged at one end of the intermediate plane 8.

Preferably, the intermediate plane 8 comprises sliding means 17 coupled to the framing 2 by shifting along the direction of sliding 9.

In more detail, the sliding means 17 comprise at least one pair of rectilinear guiding elements 18 associated with the first longitudinal elements 11a in a sliding manner.

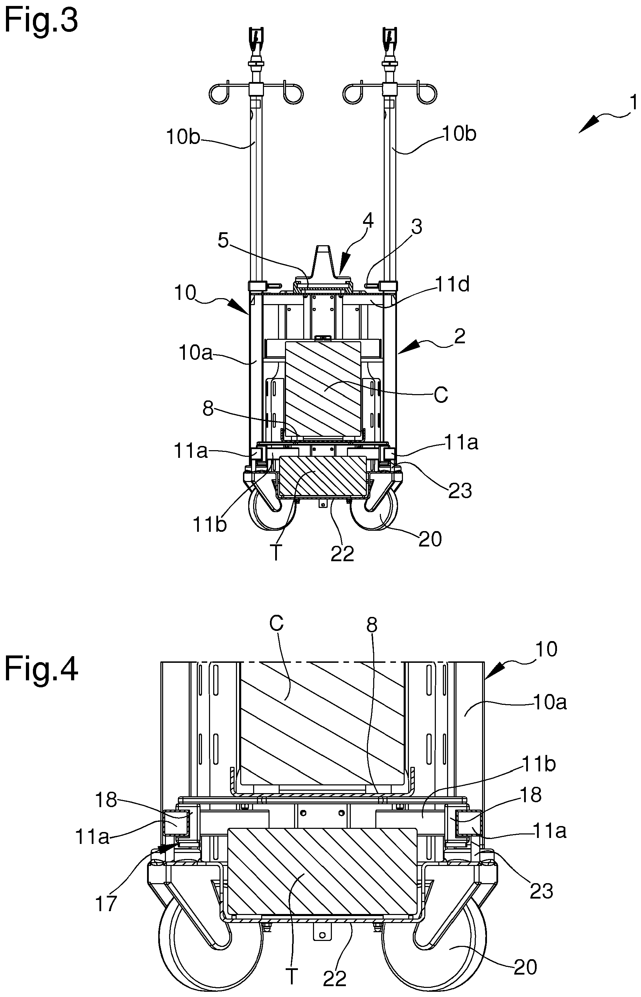

As can be seen from Figure 4, the guiding elements 18 are substantially C-shaped so as to operate in conjunction with the similar profile of the first longitudinal elements 11a and are connected to each other by means of a connecting plate 19. The guiding elements 18 are then fitted on the first longitudinal elements 11a at their inner side, that is, the side facing the other first longitudinal element 11a.

The guiding elements 18 are arranged at the point where the end of the intermediate plane 8 opposite the clamping elements 15a is located.

Appropriately, the sliding means 17, and in particular the guiding elements 18, are adapted to interact with the vertical elements 10 as a result of the shift of the intermediate plane 8 along the direction of sliding 9 towards the maintenance position. The interaction between the sliding means 17 and the vertical elements 10 defines the end-of-stroke position of the intermediate plane 8 along the direction of sliding 9.

In the preferred embodiment shown in the figures, the intermediate plane 8 also comprises a housing compartment 21 of an auxiliary unit, such as of the type of a power supply unit, connectable to at least one of either the biomedical device O,P or the heat exchanger C.

Preferably, the equipment 1 comprises at least one containment body 22 open at the top, which is associated with the framing 2 and adapted to contain a voltage stabilizer T. More particularly, the intermediate plane 8 is arranged on top of the containment body 22 in the working position and is displaced with respect thereto in the maintenance position so as to allow the access from the outside to the containment body itself.

The result, therefore, is that when the intermediate plane 8 is brought to the maintenance position, the voltage stabilizer T is easily accessible from the outside.

As can be seen from Figures 3 and 4, the containment body 22 is locked together with the first longitudinal elements 11a by means of relevant connecting walls 23 and the guiding elements 18 slide along the first longitudinal elements 11a within the space placed between the connecting walls 23. The connecting walls 23 are arranged, in use, substantially vertical.

Preferably, the equipment 1 comprises at least one supporting element 24 of at least one cylinder B containing an aeriform fluid, e.g., oxygen, associated with the framing 2 in a removable manner.

The supporting element 24 then defines a housing seat intended to receive a cylinder B and comprises removable coupling means 26, 27 to the framing 2.

Advantageously, the coupling means 26,27 comprise first coupling means 26 of the type of grippers adapted to engage, in a removable manner, with one of either the second longitudinal elements 11c or of the second transverse elements 11d.

In more detail, the first coupling means 26 comprise at least one upper gripping element 26a, a lower gripping element 26b, and control means 26c of the lower gripping element 26b operable to allow/prevent the displacement thereof with respect to the upper gripping element 26a. The control means 26c are, e.g., of the type of a lever movable between an adjustment position and a clamping position wherein it allows and prevents, respectively, the displacement of the lower gripping element 26b with respect to the upper gripping element 26a.

Preferably, the coupling means 26,27 also comprise second coupling means 27 adapted to operate in conjunction with the first lattice 12.

More particularly, the second coupling means 27 comprise at least one abutment element 27a adapted to lean on one of either the first longitudinal elements 11a or the first transverse elements 11b.

Appropriately, the second coupling means 27 comprise elastic means 27b, e.g. of the type of one or more springs, associated with the abutment element 27a to allow it to be displaced along a direction of adjustment 27c as a result of the interaction with one of the first elements 11a,11b. The elastic means 27b thus allow the position of the abutment element 27a to be varied along the direction of adjustment 27c depending on the size of the first elements 11a,11b with which it interacts. In other words, the abutment element 27a as a result of the interaction with the upper edge of one of the first elements 11a,11b causes the elastic means 27b to compress or expand.

Advantageously, at least one of the first transverse elements 11b comprises a cavity 28 and the abutment element 27a comprises a through-hole 29 which can be positioned in alignment with the cavity 28. The second coupling means 27 then comprise at least one fastening element 30 insertable in a removable manner within the through-hole 29 and within the cavity 28 when aligned with each other. The fastening element 30 allows, therefore, the abutment element 27a to be solidly fastened to the relevant first transverse element 11b in a manner that prevents the accidental removal thereof and facilitates the transport thereof.

On the other hand, the first longitudinal elements 11a have no cavity for the housing of the fastening element 30.

Therefore, the supporting element 24 can only be solidly constrained to the first transverse elements 11b; this is because the positioning at the point where the longitudinal elements 11a,11c are located would result in an unbalancing of the framing 2 and, therefore, would not allow safe movement of the equipment 1. The positioning of the supporting element 24 at the point where the longitudinal elements 11a,11c are located is therefore permitted but only for the static use of the equipment 1, whereas when the latter is to be moved, positioning at where the transverse elements 11b,11d are located and, in particular, anchoring to the first longitudinal elements 11a is required.

In the preferred embodiment shown in the Figures, the equipment 1 comprises at least one gripping element 14, associated with the framing 2 and arranged at the point where one of the second transverse elements 11d is located. More particularly, the gripping element 14 comprises a fixed portion 14a and a movable portion 14b associated with the framing 2 in a revolving manner and movable between a transport position, wherein it is directed parallel to the second transverse elements 11d and operates in conjunction with the fixed portion 14a, and a release position, wherein it is directed substantially parallel to the second longitudinal elements 11c and is disengaged from the fixed portion 14a. More particularly, the movable portion 14b and the fixed portion 14a are associated with the movable body 10b of a relevant vertical element 10, so that the height thereof can be varied as required. As can be seen from the figures, the fixed portion 14a defines a seat for housing the movable portion 14b in the transport position.

In the transport position, the movable portion 14b is thus arranged transversely to the direction of insertion/extraction 6, so as to hinder the movement of the equipment for the support of the biomedical devices 50.

It has in practice been ascertained that the described invention achieves the intended objects, and in particular the fact is emphasized that the equipment according to the invention allows for easy and practical maintenance of the transported devices, especially the heat exchanger, without the need to remove the latter from the equipment itself.

In fact, the possibility of moving the intermediate plane in a sliding manner allows easy access to the device placed thereon in the maintenance position.

At the same time, the displacement of the intermediate plane to the maintenance position also provides easy access to the containment body below.

Again, the supporting body of the oxygen cylinder can be easily displaced, thanks to the coupling means, to the most convenient position from time to time. At the same time, the supporting body of the oxygen cylinder can only be solidly fastened to the first transverse elements, since they are provided with the cavities for housing the fastening element, so that it can be safely moved together with the framing, so as to avoid the risk of tipping over during transport due to incorrect positioning where the longitudinal elements of the framing itself are located.

## Claims

1. Equipment (1) for the transport of biomedical devices, comprising:
- a load-bearing framing (2);
- movement means (20) of said framing (2) with respect to a resting surface (S);
- at least one supporting plane (3) associated with said framing (2) and provided with removable anchoring means (4) of at least one biomedical device (O, P);
wherein said framing (2) comprises a plurality of load-bearing elements (10,11) having an elongated shape and associated with each other, of which a plurality of vertical elements (10) and a plurality of horizontal elements (11) interconnected with each other,
the equipment (1) comprising at least one intermediate plane (8) for the support of an additional device (C) intended to be interconnected to said biomedical device (O, P),
**characterized by** the fact that said intermediate plane (8) is associated movable by shifting with said framing (2) along a direction of sliding (9) between a working position, wherein it is positioned inferiorly to said supporting plane (3), and a maintenance position, wherein it is shifted with respect to the working position along said direction of sliding (9) by arranging itself at least partly beyond the overall dimensions defined by said framing (2) and wherein it is associated cantilevered with said framing (2),
wherein the equipment (1) comprises removable clamping means (15) of said intermediate plane (8) to said framing (2) in said working position,
and by the fact that said intermediate plane (8) is constrained to said framing (2) in said maintenance position,
the intermediate plane (8), in the maintenance position, having an extremity portion associated with said framing (2) and the opposite extremity portion arranged outside of the framing (2) and disengaged therefrom.

2. Equipment (1) according to claim 1, **characterized by** the fact that said intermediate plane (8) is substantially vertically aligned with said supporting plane (3) in the working position and is vertically offset with respect thereto in the maintenance position.

3. Equipment (1) according to claim 1 or 2, **characterized by** the fact that said clamping means (15) comprise at least one pair of clamping elements (15a) locked together with said intermediate plane (8) and at least one pair of seats (15b) associated with said framing (2) and adapted to receive said clamping elements (15a) in the working position, thus preventing the displacement thereof along said direction of sliding (9), said clamping elements (15a) being able to be lifted up to exit said seats (15b) so as to allow the displacement of the intermediate plane (8) towards said maintenance position.

4. Equipment (1) according to one or more of the preceding claims, **characterized by** the fact that said intermediate plane (8) comprises sliding means (17) coupled to said framing (2) by shifting.

5. Equipment (1) according to claim 4, **characterized by** the fact that said framing (2) comprises at least a first lattice (12) supporting said intermediate plane (8) and provided with a pair of first longitudinal elements (11a), which develop parallel to said direction of sliding (9), and with a pair of first transverse elements (11b), and by the fact that said sliding means (17) comprise at least one pair of rectilinear guiding elements (18) associated with said first longitudinal elements (11a) in a sliding manner.

6. Equipment (1) according to claim 5, **characterized by** the fact that said sliding means (17) are adapted to interact with said vertical elements (10) as a result of the shift of said intermediate plane (8) along said direction of sliding (9) towards the maintenance position, the interaction between said sliding means (17) and said vertical elements (10) defining the end-of-stroke position of said intermediate plane (8) along said direction of sliding (9).

7. Equipment (1) according to one or more of the preceding claims, **characterized by** the fact that said intermediate plane (8) comprises at least one housing compartment (21) of an auxiliary unit connectable to at least one of either said biomedical device (O, P) or said additional device (C).

8. Equipment (1) according to one or more of the preceding claims, **characterized by** the fact that it comprises at least one containment body (22) open at the top, which is associated with said framing (2) and adapted to contain a voltage stabilizer (T), wherein said intermediate plane (8) is arranged on top of said containment body (22) in the working position and is displaced with respect thereto in the maintenance position thus allowing the access from the outside to said containment body (22).

9. Equipment (1) according to claim 8 as dependent on claim 5, **characterized by** the fact that said containment body (22) is locked together with said first longitudinal elements (11a) by means of relevant connecting walls (23) and by the fact that said guiding elements (18) slide along said first longitudinal elements (11a) within the space placed between said connecting walls (23).

10. Equipment (1) according to one or more of the preceding claims, **characterized by** the fact that it comprises a supporting element (24) of at least one cylinder (B) containing an aeriform fluid associated with said framing (2) in a removable manner.

11. Equipment (1) according to claim 10, **characterized by** the fact that said supporting element (24) comprises removable coupling means (26, 27) to said framing (2).

12. Equipment (1) according to claim 11, **characterized by** the fact that said framing (2) comprises at least one second lattice (13) adapted to support said supporting plane (3) and provided with a pair of second longitudinal elements (11c) and with a pair of second transverse elements (11d), and by the fact that said coupling means (26, 27) comprise first coupling means (26) of the type of grippers adapted to engage with one of either said second longitudinal elements (11c) or of said second transverse elements (11d) in a removable manner.

13. Equipment (1) according to claim 12, **characterized by** the fact that said coupling means (26, 27) comprise second coupling means (27) adapted to operate in conjunction with said first lattice (12).

14. Equipment (1) according to claim 12, **characterized by** the fact that it comprises at least one gripping element (14) associated with said framing (2) and arranged at the point where one of said second transverse elements (11d) is located, and by the fact that said gripping element (14) comprises a fixed portion (14a) and a movable portion (14b) associated with said framing (2) in a revolving manner and movable between a transport position, wherein it is oriented parallel to said second transverse elements (11d) and operates in conjunction with said fixed portion (14a) and a release position, wherein it is oriented substantially parallel to said second longitudinal elements (11c) and is disengaged from said fixed portion (14a).

## Patentansprüche

1. Vorrichtung (1) zum Transport biomedizinischer Geräte, umfassend:
- einen tragenden Rahmen (2);
- Bewegungsmittel (20) des Rahmens (2) relativ zu einer Auflagefläche (S);
- zumindest eine dem Rahmen (2) zugeordnete Auflagefläche (3), die abnehmbare Verankerungsmittel (4) für zumindest ein biomedizinisches Gerät (O, P) aufweist;
wobei der Rahmen (2) eine Vielzahl von tragenden Elementen (10, 11) mit länglicher Form umfasst, die einander zugeordnet sind, darunter eine Vielzahl vertikaler Elemente (10) und eine Vielzahl horizontaler Elemente (11), die miteinander verbunden sind,
wobei die Vorrichtung (1) zumindest eine Zwischenebene (8) zur Aufnahme eines zusätzlichen Geräts (C) umfasst, die dazu angeordnet ist, mit dem biomedizinischen Gerät (O, P) verbunden zu werden,
**dadurch gekennzeichnet, dass** die Zwischenebene (8) dem Rahmen (2) beweglich zugeordnet ist durch Verschieben entlang einer Verschieberichtung (9) zwischen einer Arbeitsposition, in der sie unterhalb der Auflagefläche (3) angeordnet ist,
und einer Wartungsposition, in der sie gegenüber der Arbeitsposition entlang der Verschieberichtung (9) verschoben ist, indem sie sich zumindest teilweise über die durch den Rahmen (2) definierten Gesamtabmessungen hinaus erstreckt,
und in der sie dem Rahmen (2) auskragend zugeordnet ist,
wobei die Vorrichtung (1) abnehmbare Befestigungsmittel (15) der Zwischenebene (8) an dem Rahmen (2) in der Arbeitsposition aufweist,
und dadurch, dass die Zwischenebene (8) in der Wartungsposition an dem Rahmen (2) fixiert ist,
wobei die Zwischenebene (8) in der Wartungsposition einen Endabschnitt aufweist, der dem Rahmen (2) zugeordnet ist, und der gegenüberliegende Endabschnitt außerhalb des Rahmens (2) angeordnet und von diesem gelöst ist.

2. Vorrichtung (1) nach Anspruch 1, **dadurch gekennzeichnet, dass** die Zwischenebene (8) in der Arbeitsposition im Wesentlichen vertikal zur Auflagefläche (3) angeordnet ist und in der Wartungsposition vertikal gegenüber dieser versetzt ist.

3. Vorrichtung (1) nach Anspruch 1 oder 2, **dadurch gekennzeichnet, dass** die Befestigungsmittel (15) zumindest ein Paar Befestigungselemente (15a) umfasst, die mit der Zwischenebene (8) verriegelt sind,
und zumindest ein Paar an Sitzen (15b), die dem Rahmen (2) zugeordnet und dazu angeordnet sind,
die Befestigungselemente (15a) in der Arbeitsposition aufzunehmen, um deren Verschiebung entlang der Verschieberichtung (9) zu verhindern, wobei die Befestigungselemente (15a) anhebbar sind, um aus den Sitzen (15b) auszutreten, um die Verschiebung der Zwischenebene (8) in Richtung der Wartungsposition zu ermöglichen.

4. Vorrichtung (1) nach einem oder mehreren der vorhergehenden Ansprüche, **dadurch gekennzeichnet, dass** die Zwischenebene (8) eine Gleitvorrichtungen (17) umfasst, die verschiebbar mit dem Rahmen (2) verbunden ist.

5. Vorrichtung (1) nach Anspruch 4, **dadurch gekennzeichnet, dass** der Rahmen (2) zumindest ein erstes Gitter (12) umfasst, das die Zwischenebene (8) trägt und ein Paar erster Längselemente (11a) umfasst, die sich parallel zur Verschieberichtung (9) erstrecken, und ein Paar erster Querelemente (11b) umfasst, und dass die Gleitvorrichtungen (17) zumindest ein Paar geradliniger Führungselemente (18) umfasst, die den ersten Längselementen (11a) gleitend zugeordnet sind.

6. Vorrichtung (1) nach Anspruch 5, **dadurch gekennzeichnet, dass** die Gleitvorrichtungen (17) ausgebildet sind, um mit den vertikalen Elementen (10) zusammenzuwirken infolge der Verschiebung der Zwischenebene (8) entlang der Verschieberichtung (9) in Richtung zur Wartungsposition, wobei das Zusammenwirken zwischen den Gleitvorrichtungen (17) und den vertikalen Elementen (10) die Endlage der Zwischenebene (8) entlang der Verschieberichtung (9) festlegt.

7. Vorrichtung (1) nach einem oder mehreren der vorhergehenden Ansprüche, **dadurch gekennzeichnet, dass** die Zwischenebene (8) zumindest eine Gehäuseeinheit (21) einer Hilfseinheit umfasst, die mit zumindest einem der beiden Geräte, nämlich dem biomedizinischen Gerät (O, P) oder dem zusätzlichen Gerät (C), verbindbar ist.

8. Vorrichtung (1) nach einem oder mehreren der vorhergehenden Ansprüche, **dadurch gekennzeichnet, dass** sie zumindest einen nach oben offenen Aufnahmekörper (22) umfasst, der dem Rahmen (2) zugeordnet und zur Aufnahme eines Spannungsstabilisators (T) ausgebildet ist, wobei die Zwischenebene (8) in der Arbeitsposition auf dem Aufnahmekörper (22) angeordnet ist und in der Wartungsposition gegenüber diesem verschoben ist, um Zugang von außen zum Aufnahmekörper (22) zu ermöglichen.

9. Vorrichtung (1) nach Anspruch 8, in Abhängigkeit von Anspruch 5, **dadurch gekennzeichnet, dass** der Aufnahmekörper (22) mit den ersten Längselementen (11a) mittels entsprechender Verbindungswände (23) verriegelt ist, und dass die Führungselemente (18) entlang der ersten Längselemente (11a) innerhalb des zwischen den Verbindungswänden (23) liegenden Raums gleiten.

10. Vorrichtung (1) nach einem oder mehreren der vorhergehenden Ansprüche, **dadurch gekennzeichnet, dass** sie ein Stützelement (24) aus zumindest einem Zylinder (B) umfasst, der ein gasförmiges Fluid enthält und abnehmbar an den Rahmen (2) angekoppelt ist.

11. Vorrichtung (1) nach Anspruch 10, **dadurch gekennzeichnet, dass** das Stützelement (24) vom Rahmen (2) abnehmbare Kopplungsmittel (26, 27) aufweist.

12. Vorrichtung (1) nach Anspruch 11, **dadurch gekennzeichnet, dass** der Rahmen (2) zumindest ein zweites Gitter (13) umfasst, das zum Tragen der Auflagefläche (3) ausgebildet ist
und ein Paar zweiter Längselemente (11c) und ein Paar zweiter Querelemente (11d) aufweist,
und dass die Kopplungsmittel (26, 27) erste Kopplungsmittel (26) in Form von Greifern umfassen, die dazu ausgebildet sind, lösbar mit einem der zweiten Längselemente (11c) oder einem der zweiten Querelemente (11d) gekoppelt zu werden.

13. Vorrichtung (1) nach Anspruch 12, **dadurch gekennzeichnet, dass** die Kopplungsmittel (26, 27) zweite Kopplungsmittel (27) umfassen, die so ausgebildet sind, dass sie in Verbindung mit dem ersten Gitter (12) zusammenwirken.

14. Vorrichtung (1) nach Anspruch 12, **dadurch gekennzeichnet, dass** sie zumindest ein Greifelement (14) umfasst, das dem Rahmen (2) zugeordnet und an der Stelle angeordnet ist, an der sich eines der zweiten Querelemente (11d) befindet,
und dass das Greifelement (14) einen feststehenden Abschnitt (14a) und einen beweglichen Abschnitt (14b) aufweist, der dem Rahmen (2) drehbar zugeordnet ist und
zwischen einer Transportposition, in der er parallel zu den zweiten Querelementen (11d) angeordnet ist und mit dem feststehenden Abschnitt (14a) zusammenwirkt, und einer Freigabeposition bewegbar ist,
in der er im Wesentlichen parallel zu den zweiten Längselementen (11c) angeordnet und vom feststehenden Abschnitt (14a) gelöst ist.

## Revendications

1. - Équipement (1) pour le transport de dispositifs biomédicaux, comprenant :
- un bâti porteur de charge (2) ;
- des moyens de déplacement (20) dudit bâti (2) par rapport à une surface de pose (S) ;
- au moins un plan de support (3) associé audit bâti (2) et comportant des moyens d'ancrage amovibles (4) d'au moins un dispositif biomédical (O, P) ;
dans lequel ledit bâti (2) comprend une pluralité d'éléments porteurs de charge (10, 11) ayant une forme allongée et associés les uns aux autres, dont une pluralité d'éléments verticaux (10) et une pluralité d'éléments horizontaux (11) interconnectés les uns aux autres,
l'équipement (1) comprenant au moins un plan intermédiaire (8) pour le support d'un dispositif supplémentaire (C) destiné à être interconnecté audit dispositif biomédical (O, P),
**caractérisé par le fait que** ledit plan intermédiaire (8) est associé de manière mobile par décalage audit bâti (2) le long d'une direction de coulissement (9) entre une position de travail, dans laquelle il est positionné au-dessous dudit plan de support (3), et une position de maintenance, dans laquelle il est décalé par rapport à la position de travail le long de ladite direction de coulissement (9) en étant disposé lui-même au moins partiellement au-delà de l'encombrement défini par ledit bâti (2) et dans laquelle il est associé en porte-à-faux audit bâti (2),
dans lequel l'équipement (1) comprend des moyens de serrage amovibles (15) dudit plan intermédiaire (8) audit bâti (2) dans ladite position de travail, et
**par le fait que** ledit plan intermédiaire (8) est contraint sur ledit bâti (2) dans ladite position de maintenance,
le plan intermédiaire (8), dans la position de maintenance, présentant une partie d'extrémité associée audit bâti (2) et la partie d'extrémité opposée disposée à l'extérieur du bâti (2) et désengagée de celui-ci.

2. - Équipement (1) selon la revendication 1, **caractérisé par le fait que** ledit plan intermédiaire (8) est sensiblement aligné verticalement avec ledit plan de support (3) dans la position de travail et est décalé verticalement par rapport à celui-ci dans la position de maintenance.

3. - Équipement (1) selon la revendication 1 ou 2, **caractérisé par le fait que** lesdits moyens de serrage (15) comprennent au moins une paire d'éléments de serrage (15a) verrouillés conjointement avec ledit plan intermédiaire (8) et au moins une paire de sièges (15b) associés audit bâti (2) et conçus pour recevoir lesdits éléments de serrage (15a) dans la position de travail, empêchant ainsi leur déplacement le long de ladite direction de coulissement (9), lesdits éléments de serrage (15a) étant aptes à être soulevés pour sortir desdits sièges (15b) de manière à permettre le déplacement du plan intermédiaire (8) vers ladite position de maintenance.

4. - Équipement (1) selon une ou plusieurs des revendications précédentes, **caractérisé par le fait que** ledit plan intermédiaire (8) comprend des moyens de coulissement (17) couplés audit bâti (2) par décalage.

5. - Équipement (1) selon la revendication 4, **caractérisé par le fait que** ledit bâti (2) comprend au moins un premier treillis (12) supportant ledit plan intermédiaire (8) et comportant une paire de premiers éléments longitudinaux (11a), qui s'étendent parallèlement à ladite direction de coulissement (9), et une paire de premiers éléments transversaux (11b), et **par le fait que** lesdits moyens de coulissement (17) comprennent au moins une paire d'éléments de guidage rectilignes (18) associés auxdits premiers éléments longitudinaux (11a) de manière coulissante.

6. - Équipement (1) selon la revendication 5, **caractérisé par le fait que** lesdits moyens de coulissement (17) sont conçus pour interagir avec lesdits éléments verticaux (10) à la suite du décalage dudit plan intermédiaire (8) le long de ladite direction de coulissement (9) vers la position de maintenance, l'interaction entre lesdits moyens de coulissement (17) et lesdits éléments verticaux (10) définissant la position de fin de course dudit plan intermédiaire (8) le long de ladite direction de coulissement (9).

7. - Équipement (1) selon une ou plusieurs des revendications précédentes, **caractérisé par le fait que** ledit plan intermédiaire (8) comprend au moins un compartiment de logement (21) d'une unité auxiliaire apte à être reliée à au moins l'un parmi ledit dispositif biomédical (O, P) ou ledit dispositif supplémentaire (C).

8. - Équipement (1) selon une ou plusieurs des revendications précédentes, **caractérisé par le fait qu'**il comprend au moins un corps de confinement (22) ouvert sur le dessus, qui est associé audit bâti (2) et conçu pour contenir un stabilisateur de tension (T), dans lequel ledit plan intermédiaire (8) est disposé au-dessus dudit corps de confinement (22) dans la position de travail et est déplacé par rapport à celui-ci dans la position de maintenance, permettant ainsi l'accès depuis l'extérieur audit corps de confinement (22).

9. - Équipement (1) selon la revendication 8 en dépendance de la revendication 5, **caractérisé par le fait que** ledit corps de confinement (22) est verrouillé conjointement avec lesdits premiers éléments longitudinaux (11a) au moyen de parois de liaison (23) correspondantes, et **par le fait que** lesdits éléments de guidage (18) coulissent le long desdits premiers éléments longitudinaux (11a) dans l'espace situé entre lesdites parois de liaison (23).

10. - Équipement (1) selon une ou plusieurs des revendications précédentes, **caractérisé par le fait qu'**il comprend un élément de support (24) d'au moins un cylindre (B) contenant un fluide aériforme, associé audit bâti (2) de manière amovible.

11. - Équipement (1) selon la revendication 10, **caractérisé par le fait que** ledit élément de support (24) comprend des moyens d'accouplement amovibles (26, 27) audit bâti (2).

12. - Équipement (1) selon la revendication 11, **caractérisé par le fait que** ledit bâti (2) comprend au moins un second treillis (13) conçu pour supporter ledit plan de support (3) et comportant une paire de seconds éléments longitudinaux (11c) et une paire de seconds éléments transversaux (11d), et **par le fait que** lesdits moyens d'accouplement (26, 27) comprennent des premiers moyens d'accouplement (26) du type pinces conçus pour s'engager avec l'un parmi lesdits seconds éléments longitudinaux (11c) ou lesdits seconds éléments transversaux (11d) de manière amovible.

13. - Équipement (1) selon la revendication 12, **caractérisé par le fait que** lesdits moyens d'accouplement (26, 27) comprennent des seconds moyens d'accouplement (27) conçus pour fonctionner conjointement avec ledit premier treillis (12).

14. - Équipement (1) selon la revendication 12, **caractérisé par le fait qu'**il comprend au moins un élément de préhension (14) associé audit bâti (2) et disposé à l'emplacement où l'un desdits seconds éléments transversaux (11d) est situé, et **par le fait que** ledit élément de préhension (14) comprend une partie fixe (14a) et une partie mobile (14b) associée audit bâti (2) de manière rotative et mobile entre une position de transport, dans laquelle elle est orientée parallèlement auxdits seconds éléments transversaux (11d) et fonctionne conjointement avec ladite partie fixe (14a), et une position de libération, dans laquelle elle est orientée sensiblement parallèlement auxdits seconds éléments longitudinaux (11c) et est désengagée de ladite partie fixe (14a).
